(19) Europäisches Patentamt
European Patent Office
Office européen des brevets

(11) Publication number: **0 002 310**
**B1**

(12) **EUROPEAN PATENT SPECIFICATION**

(45) Date of publication of patent specification: **24.06.81**

(21) Application number: **78300485.6**

(22) Date of filing: **11.10.78**

(51) Int. Cl.[3]: **C 07 D 249/22,**
C 07 D 249/16,
A 61 K 31/41

(54) **4,9-Dihydro-4,9-dioxo-1H-naphtho(2,3-d) triazoles, their preparation and antiallergic compositions containing them.**

(30) Priority: **10.11.77 GB 4676577**
**10.11.77 GB 4676777**
**22.07.78 GB 3076678**

(43) Date of publication of application:
**13.06.79 Bulletin 79/12**

(45) Publication of the grant of the European patent:
**24.06.81 Bulletin 81/25**

(84) Designated Contracting States:
**BE CH DE FR GB LU NL SE**

(56) References cited:
**US - A - 2 879 274**
**US - A - 3 198 795**
**US - A - 3 294 812**

**JOURNAL OF THE AMERICAN CHEMICAL SOCIETY, volume 37, 1935, by L. F. FIESER and E. L. MARTIN, pages 1844—1849**

(73) Proprietor: **BEECHAM GROUP LIMITED**
**Beecham House Great West Road**
**Brentford, Middlesex (GB)**

(72) Inventor: **Buckle, Derek Richard**
**18, Hillfield Road**
**Redhill Surrey (GB)**
Inventor: **Smith, Harry**
**Thronthwaite Cottage Copsale Road**
**Maplehurst Nr. Horsham Sussex (GB)**

(74) Representative: **Dawson, Hugh Bainforde et al,**
**Beecham Pharmaceuticals Great Burgh Yew Tree Bottom Road**
**Epsom Surrey KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

4,9-Dihydro-4,9-dioxo-1-H-naphtho[2,3-d]triazoles, their preparation and antiallergic compositions containing them

This invention relates to pharmaceutical compositions containing a class of 4,9-dihydro-4,9-dioxo-1H-naphtho[2,3-d]-triazoles, which are useful in the treatment of allergic diseases, to certain novel compounds within this class, and to a process for the preparation of these novel compounds.

US Patent No. 3,294,812 discloses that the compound of formula (A):

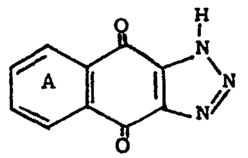

(A)

may be used as in intermediate in the preparation of fluorescent pigments. It is also suggested in this Patent that the ring A can be substituted by lower alkyl, lower alkoxy, halogen (eg chloro), nitro or lower alkanamido, but no examples of such substitution are given.

US Patent No. 2,879,274 discloses the compound of formula (A), and also its 6-methyl analogue, as intermediates in the preparation of dyestuffs.

US Patent No. 3,198,795 discloses compounds of formula (B):

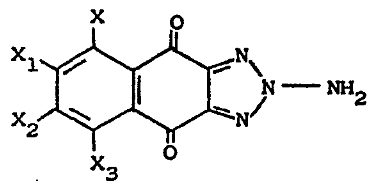

(B)

wherein X, $X_1$, $X_2$ and $X_3$ are a range of substituents, as pigments or colorants for plastics.

The aforesaid compound (A) has been a known compound for many years, and listed below are a number of literature references each of which discloses a different by which the compound may be prepared. The compounds is named 4,9-dihydro-4,9-dioxo-1H-naphtho[2,3-d]triazole.

K. Fries, R. Walter and K. Schilling, *Annalen 576* 248 (1935). L. F. Fieser and E. L. Martin, *J. Amer. Chem. Soc. 57* 1844 (1935). W. L. Mosby and M. L. Silva, *J. Chem. Soc.* 1003 (1965).

It has never been reported or suggested in the literature that the compounds referred to above have any type of pharmaceutical activity.

In complete contrast to the references discussed above, the present invention relates to anti-allergy drugs.

It is generally recognised that certain cells eg mast cells, are activated by antibody-antigen combinations and release substances such as histamine and SRS—A, which mediate an allergic response. We have discovered that 4,9-dihydro-4,9-dioxo-1H-naphtho[2,3-d]-triazoles of formula (I):

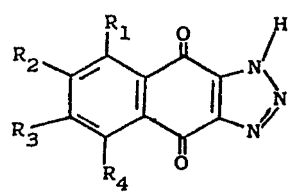

(I)

and pharmaceutically acceptable salts thereof, wherein $R_1$, $R_2$, $R_3$ and $R_4$ which may be the same or different, represent hydrogen, halogen, nitro, $C_{1-6}$ alkyl or $C_{1-6}$ alkoxy, or any adjacent two of $R_1$ to $_4$ taken together represent an alkylene group containing from 3 to 5 carbon atoms or a 1,4-buta-1,3-dienylene group, inhibit this type of antigen-induced response in mammals, and are therefore of value in the prophylaxis of diseases in which the symptoms are controlled by mediators of the allergic response. Examples of such diseases include bronchial asthma, rhinitis, hayfever and allergic eczema.

Accordingly, in its broadest aspect, the present invention provides a pharmaceutical composition, which composition comprises a compound of the formula (I), as hereinbefore defined, or a pharmaceutically acceptable salt thereof, and a pharmaceutically acceptable carrier.

Compounds of formula (I) may be administered topically or systematically. In accordance with usual pharmaceutical procedure, the active material will be purified so as to contain minimum amounts of by-products or other impurities.

Topical formulatioans for administration to the skin include lotions and creams. Topical formulations for administration to the respiratory tract include solutions for application *via* a nebulizer or as an aerosol, snuffs and microfine insufflatable powders. The active ingredient in an insufflatable

powder has a small particle size ie less than 50 microns and preferably less than 10 microns. The active material is co-presented with a solid carrier such as lactose which has a particle size of less than 50 microns. Insufflatable compositions in particular will be rendered substantially free of microbial contaminant.

Systemic administration may be achieved by rectal, oral or parenteral administration. A typical suppository formulation comprises the active compound with a binding and/or lubricating agent such as gelatin or cocoa butter or other low melting vegetable waxes or fats. Typical parenteral compositions comprise a solution or suspension of the active material in a sterile aqueous carrier or parenterally acceptable oil.

Compounds of formula (I) which are active when given orally may be compounded in the form of syrups, tablets, capsules and lozenges. A syrup formulation will generally consist of a suspension or solution of the compound in a suitable liquid carrier such as ethyl alcohol, glycerine or water with a flavouring or colouring agent. Where the composition is in the form of a capsule, the solid in granular form optionally with a binding agent is encapsulate in an edible shell eg of gelatin. Where the composition is in the form of a tablet, any suitable pharmaceutical carrier routinely used for preparing solid formulations may be used. Examples of such carriers include magnesium stearate, starch, lactose, glucose, sucrose, rice flour and chalk. Preferably the composition is in unit dose form such a pill, capsule or metered aerosol so that the patient may administer to himself a single dose.

Where appropriate, small amouants of anti-asthmatics and bronchodilators for example sympathomimetic amines such as isoprenaline, isoetharine, salbutamol, phenylephrine and ephedrine; xanthine derivatives such as theophylline and aminophylline; and corticosteroids such as prednisolone and adrenal stimulants such as ACTH may be included. As is common practice, the compositions will usually be accompanied by written or printed directions for use in the medical treatment concerned, in this case as an anti-allergic agent for treatment of, for example, asthma, hayfever or rhinitis.

The compositions are suitably prepared in accordance with standard pharmaceutical practice.

In the compounds of formula (I), examples of suitable alkyl groups include methyl, ethyl and *n*-propyl.

Examples of suitable alkoxy groups include methoxy, ethoxy and *n*-propoxy.

Examples of suitable halogens include fluorine and chlorine.

The alkylene groups which any two of $R_1$ to $R_4$ may together represent are propylene, butylene and pentylene.

Where compounds of formula (I) are highly substituted, it is appreciated that substituents $R_1$ to $R_4$ are selected for steric compatability.

Within the compounds of formula (I) there are a variety of interesting sub-groups.

One such group is that in which at least one of $R_1$ to $R_4$ is hydrogen and the remainder are as previously defined. An example of such a compound is 4,9-dihydro-6,7-dimethyl-5-nitro-4,9-dioxo-1H-naphtho[2,3-d]-triazole. A further group is one where two of $R_1$ to $R_4$ are hydrogen and the remainder are as previously defined. An example of such a compound is 4,9-dihydro-5,6-dimethyl-4,9-dioxo-1H-naphtho-[2,3-d]-triazole. One preferred sub-group of compounds of formula (I) is that in which $R_1$ and $R_4$ are hydrogen and $R_2$ and $R_3$, which may be the same or different, represent methyl, ethyl or *n*-propyl.

An example of one such compound is: 4,9-dihydro-6,7-dimethyl-4,9-dioxo-1H-naphtho-[2,3-d]-triazole, which together with its sodium salt, is the preferred compound for use in the compositions of this invention.

It is believed that the majority of the compounds of the formula (I) are novel, and as such form an important part of this invention.

More specifically the invention provides, as novel compounds, compounds of formula (I)':

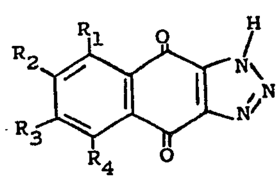

(I)'

and pharmaceutically acceptable salts thereof, wherein $R_1$ and $R_4$ are the same or different and represent hydrogen, halogen, nitro, $C_{1-6}$ alkyl or $C_{1-6}$ alkoxy; and $R_2$ and $R_3$ are the same or different and represent halogen, nitro, $C_{1-6}$ alkyl or $C_{1-6}$ alkoxy; or $R_2$ and $R_3$ taken together represent an alkylene group containing from 3 to 5 carbon atoms or a 1,4-buta-1,3-dienylene group.

Suitable and preferred examples of the variable groups in formula (I)', and of sub-groups and compounds within formula (I)', are as hereinbefore described in relation to formula (I).

The triazole moiety of the compounds of formula (I) has an acidic hydrogen, and accordingly may form salts. Examples of pharmaceutically acceptable salts falling within the scope of this invention include aluminium, alkali metal and alkaline earth metal salts such as the sodium potassium and magnesium salt; and salts with organic bases such as amines or amino compounds including physiologically active amines such as (—) epherine. The sodium and ephedrine salts (—) are preferred.

The invention also provides a compound of the formula (I) as defined for use in the treatment of allergies.

The invention also provides methods for preparing the novel compounds of the invention, and their salts.

The first method comprises oxidizing a naphthotriazole of formula (II):

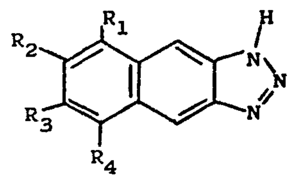

(II)

wherein $R_1$, $R_2$, $R_3$ and $R_4$ are as defined with reference to formula (I)' with a powerful oxidizing agent, and thereafter optionally converting the compound of formula (I)' into a pharmaceutically acceptable salt.

Suitable oxidizing agents include chromium trioxide and chromic acid. The method is carried out in a manner analogous to that described by K. Fries, R. Walter and K. Schilling, *Annalen 576,* 248, (1935); for the preparation of 4,9-dihydro-4,9-dioxo-1H-naphtho-[2,3-d]-triazole.

A second method comprises deamination of a 2-amino naphtho triazole (III):

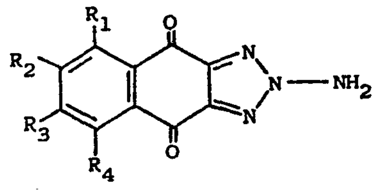

(III)

wherein $R_1$, $R_2$, $R_3$, and $R_4$ are as defined with reference to formula (I) above, with nitrous acid, and thereafter optionally converting the compound of formula (I) into a pharmaceutically acceptable salt thereof.

The method is carried out in a manner analogous to that described by W. L. Mosby and M. L. Silva, *J. Chem. Soc.,* 1003, (1965), for the preparation of 4,9-dihydro-4,9-dioxo-1H-naphtho[2,3-d]-triazole.

However, we have found that compounds of formula (I) above are most conveniently made by a process which comprises reacting a compound of formula (IV):—

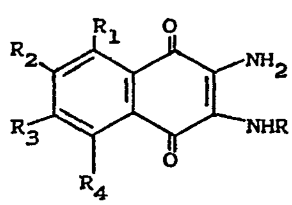

(IV)

wherein $R_1$, $R_2$, $R_3$ and $R_4$ are as defined with reference to formula (I) and R is hydrogen or an acyl group, with nitrous acid, and thereafter optionally converting the compound of formula (I) into a pharmaceutically acceptable salt.

Suitable acyl groups include benzoyl and lower alkanoyl. Examples of suitable lower alkanoyl groups are acetyl, propionyl and butyryl.

The nitrous acid is most suitably generated *in situ* from an alkali metal nitrite and an acid.

Most suitably the alkali metal nitrite is sodium nitrite, and the acid is a mineral acid such as hydrochloric acid.

The reaction is carried out in a solvent which is inert to the reagents and products. Examples of such solvents include water and acetic acid.

We have found water to be most convenient.

The reaction should be carried out at room temperature or below i.e. preferably between 0°C and 25°C.

The preparation of the compounds (I) from the quinone intermediates (IV) is facilitated by reducing the quinone to the corresponding hydroquinone, (IVa):—

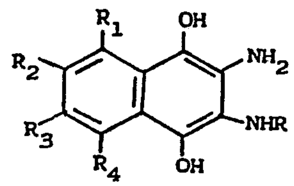

(IVa)

wherein R and $R_1$ to $R_4$ are as defined with reference to formula (IV) above and reacting the hydroquinone (IVa) with nitrous acid. These are generally more soluble in acid media than the quinones. The reduction may be carried out using any standard method for reducing quinones to hydroquinones. We have found sodium dithionite to be a most convenient reducing agent for this purpose. The hydroquinones (IVa) oxidize to the parent quinone during the reaction with nitrous acid.

Naphthotriazoles of this invention having at least one nitro substituent may be prepared by direct nitration of an appropriate triazole.

In a further aspect the invention provides a process for preparing a compound of formula (Ia):—

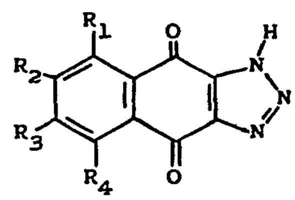

(Ia)

and pharmaceutically acceptable salts thereof; wherein $R_1$, $R_2$, $R_3$ and $R_4$ which may be the same or different represent hydrogen, halogen, nitro, lower alkyl and lower alkoxy, or any adjacent two of $R_1$ to $R_4$ taken together represent an alkylene group containing from 3 to 5 carbon atoms or a 1,4-buta-1,3-dienylene group, provided that at least one of $R_1$ to $R_4$ represent nitro which process comprises nitrating a compound of formula (Ib):—

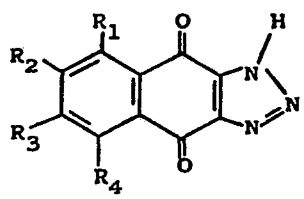

(Ib)

wherein $R_1$, $R_2$, $R_3$ and $R_4$, which may be the same or different, represent hydrogen, halogen, nitro, lower alkyl or lower alkoxy, or any adjacent two of $R_1$ to $R_4$ taken together represent an alkylene group containing from 3 to 5 carbon atoms or a 1,4-buta-1,3-dienylene group, provided that at least one of $R_1$ to $R_4$ represents hydrogen.

The reaction is carried out with a conventional nitrating agent under conventional reaction conditions. For convenience we choose to carry out the reaction with fuming nitric acid and concentrated sulphuric acid. Equally, the nitration step could be carried out with other standard reagents.

The choice of temperature at which the reaction is carried out is dependent upon the reactivity and sensitivity of the starting materials. Thus compounds which do not decompose to any significant extent may be nitrated rapidly at high temperatures for example up to 120°C, 100°C being generally convenient. More sensitive compounds require more prolonged reaction times at lower temperatures. The time for which a reaction should be allowed to proceed depends upon the starting materials, nitrating agent and temperature but may be determined by routine procedures e.g. by following the course of the reaction by thin layer chromatography.

The synthetic route to the intermediates (IV) is summarised in the following scheme in which R, $R_1$, $R_2$, $R_3$ and $R_4$ are as defined with reference to formula (IV) above, and X is chlorine or bromine and R is an acyl group as discussed above with reference to (IV).

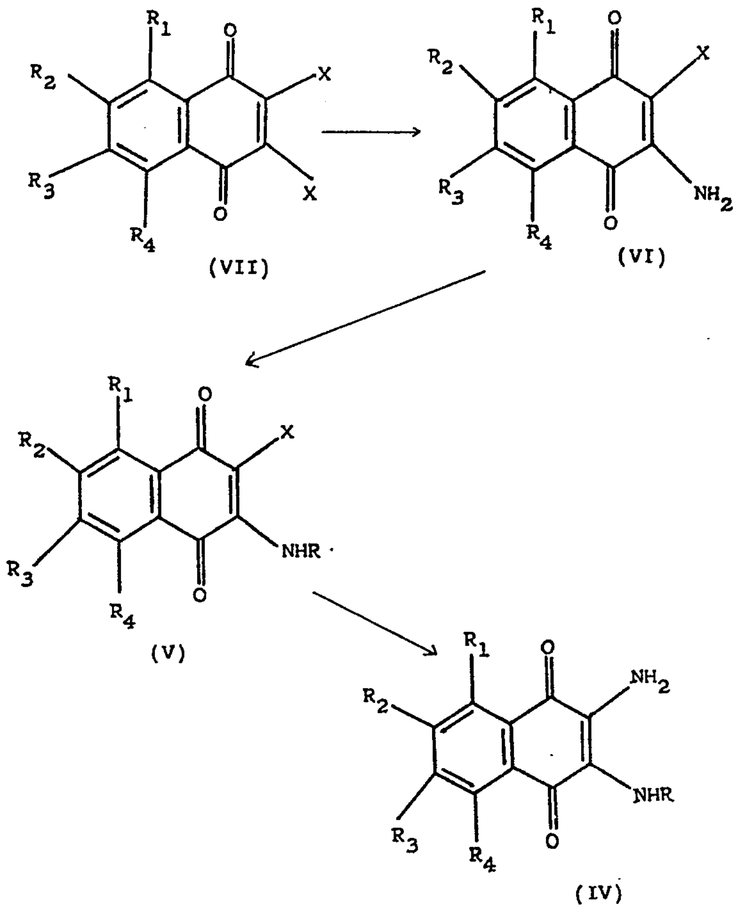

(VII) (VI) (V) (IV)

The intermediate (IV) is prepared by displacement of halogen from the corresponding 2-acylamino-3-halonaphtho-1,4-quinone (V) using ammonia and where desired thereafter removing the acyl group.

The reaction is carried out by passing dry ammonia into a solution of the compound (V) in a high boiling aprotic solvent which is inert to the reagent and products. Examples of such solvents include nitrobenzene, N,N-dimethylformamide and hexamethylphosphoramide. Preferably the solvent is nitrobenzene. Suitably the reaction is carried out at elevated temperatures (i.e. 80—180°C).

The 2-acylamino-3-halonaphtho-1,4-quinones (V) are prepared by reacting an active N-acylating derivative of benzoic or a lower alkanoic acid R —OH with a 2-amino-3-halonaphthoquinone (VI). Suitable N-acylating derivatives include the acid halide and the acid anhydride. This reaction is generally carried out in the absence of solvent in the presence of an excess of the N-acylating derivative. If desired, the reaction may be carried out in a polar organic solvent which is inert to the reagents and products. Examples of such solvents include pyridine and acetic acid.

The reaction is also suitably carried out at moderate temperature i.e. less than 100°C, room temperature being most convenient. In order to facilitate the reaction, a small amount of mineral acid may be added as a catalyst.

The 2-amino-3-halo-1,4-naphthoquinones (VI) are prepared by reacting the corresponding 2,3-dihalo-1,4-naphthoquinone (VII) with ammonia. This reaction is performed by passing dry ammonia into a solution of the compound (VII) in a high boiling aprotic organic solvent which is inert to the reagents and products, as described for the conversion of (V) to (IV) above.

Preferably the solvent is nitrobenzene. Suitably the reaction is carried out at elevated temperatures i.e. 80—180°C.

The 2,3-dihalo-1,4-naphthoquinones (VII) are prepared from the corresponding naphthoquinones by a conventional halogenation reaction.

The following Examples illustrate the invention.

Example 1

a). 2-Amino-3-bromo-6,7-dimethyl-1,4-naphthoquinone

Dry ammonia was passed through a refluxing solution of 2,3-dibromo-6,7-dimethyl-1,4-naphthoquinone (10 g; mp 237—238°C) in dry nitrobenzene (65 ml) for 30 mins. and the orange solid which separated on cooling was filtered off and washed with light petroleum. After removal of ammonium bromide with water, 6.96 g (86%) of material of mp 228°C was obtained. Recrystallisation from glacial

acetic acid gave analytically pure material of the same melting point. νmax(mull) 3400, 3300, 1680, 1610, 1585, 1570 $cm^{-1}$. δ (DMSO) 2.35 (6H,s); 7.35 (2H, exchangeable broad singlet); 7.74 (2H,s). (Found; C, 51.50; H, 3.38; N, 4.79; $C_{12}H_{10}BrNO_2$ requires; C, 51.45; H, 3.60; N, 5.00%).

b). 2-Acetamido-3-bromo-6,7-dimethyl-1,4-naphthoquinone

10 g of 2-amino-3-bromo-6,7-dimethyl-1,4-naphthoquinone were dissolved in acetic anhydride (100 ml) containing a few drops of concentrated sulphuric acid and the yellow acetyl derivative which separated was filtered off and dried to give 9.0 g of crude product. Recrystallisation from chloroformpetrol gave 7.75 g (67%) of material of mp 252°C. νmax(mull) 3210, 1685, 1665, 1600 $cm^{-1}$; δ(DMSO) 2.11 (3H,s); 2.38 (6H,s); 7.80 (1H,s); 7.85 (1H,s), 1 low field exchangeable proton. (Found; C, 50.98; H, 3.76; N, 4.30; $C_{14}H_{12}BrNO_3$ requires; C, 52.03; H, 3.74; N, 4.33%).

c). 2-Acetamido-3-amino-6,7-dimethyl-1,4-naphthoquinone

Dry ammonia was passed through a stirred refluxing solution of 2-acetamido-3-bromo-6,7-dimethyl-1,4-naphthoquinone (7.5 g) in nitrobenzene (50 ml) for 1 hr. and the mixture cooled. The red solid which separated was filtered off, washed with ether-petrol and then water to give 4.46 g (77%) of material of mp 212—216°C. Recrystallisation from ethanol raised the melting point to 216°C, νmax(mull), 3400, 3270, 1675, 1665, 1630 $cm^{-1}$. δ(DMSO) 2.05 (3H,s); 2.35 (6H,s); 6.64 (2H, exchangeable s); 7.71 (2H,s); 9.00 (1H, exchangeable s.) (Found; C, 65.07; H, 5.43; N, 10.66; $C_{14}H_{14}N_2O_3$ requires; C, 65.10; H, 5.46; N, 10.85%).

d). 4,9-dihydro-6,7-dimethyl-4,9-dioxo-1H-naphthol[2,3-d]-triazole

Sodium dithionite (2.5 g) in water (10 ml) was added to 2-acetamido-3-amino-6,7-dimethyl-1,4-naphthoquinone (1.68 g) in water (150 ml) and the stirred suspension left for 1 hr. The white hydroquinone which formed was filtered off and added to 2N HCl (50 ml). The solution was cooled to 0°C and a solution of sodium nitrite (2 g) in water (20 ml) was added over 2 hrs. The suspension which formed was stirred overnight at room temperature and the white product filtered off to give 1.34 g (84%) of material of mp 262°C (dec) which was shown to be a monohydrate, νmax(mull) 3590, 3420, 1700, 1665, 1595 $cm^{-1}$. After recrystallisation from acetone-water and drying under vacuum at 107°C, the anhydrous material was isolated, νmax(mull) 3120, 1695, 1675, 1595 $cm^{-1}$. δ(DMSO) 2.22 (6H,s); 7.56 (2H,s); 1 low field broad exchangeable proton. (Found; C, 63.20; H, 3.96; N, 18.33; $C_{12}H_9N_3O_2$ requires C, 63.44, H, 3.99; N, 18.50%).

Example 2

a). 2-Amino-3-bromo-6(7)-methyl-1,4-naphthoquinone

Dry ammonia was passed through a stirred, refluxing solution of 2,3-dibromo-6-methyl-1,4-naphthoquinone (12.3 g, mp 192°C) in dry nitrobenzene (75 ml) for 1 hr. The red solid which separated on cooling was filtered off, washed well with petroleum ether (bp. 40—60) and water and recrystallised from glacial acetic acid to give 7.5 (75%) of material of mp 173°C. (Found; C, 49.78; H, 3.23; N, 5.08; $C_{11}H_8BrNO_2$ requires; C, 49.65; H, 3.03; N, 5.26%).

b). 2-Acetamido-3-bromo-6(7)-methyl-1,4-naphthoquinone

Acetylation of 2-amino-3-bromo-6(7)-methyl-1,4-naphthoquinone (7.5 g) as described in Example 1b afforded 6.0 g (70%) of the 2-acetamido derivative of mp 192—193°C. (Found; C, 49.51; H, 3.23; Br, 25.59; N, 4.16; $C_{13}H_{10}BrNO_3$ requires; C, 50.67; H, 3.27; Br, 25.93; N, 4.54%).

c). 2-Acetamido-3-amino-6(7)-methyl-1,4-naphthoquinone

Dry ammonia was passed through a stirred refluxing solution of 2-acetamido-3-bromo-6(7)-methyl-1,4-naphthoquinone (6 g) in nitrobenzene (50 ml) for 1 hr. and the product worked up as in example 1c to give 1.7 g (40%) of title compound of mp (EtOH) 200—201°C. (Found; C, 63.80; H, 4.92; N, 11.16; $C_{13}H_{12}N_2O_3$ requires; C, 63.93; H, 4.95; N, 11.47%).

d). 4,9-Dihydro-4,9-dioxo-6-methyl-1H-naphtho[2,3-d]-triazole

1.6 g of 2-acetamido-3-amino-6(7)-methyl-1,4-naphthoquinone was converted into the title triazole by the procedure outlined in example 1d. Yield 0.75 g (51%) mp (aqueous acetone) 235° (dec.) (Found; C, 57.25; H, 3.77; N, 18.00; $C_{11}H_7N_3O_2.H_2O$ requires; C, 57.07; H, 3.92; N, 18.27%.

Example 3

a). 2,3-Dibromo-6-methoxynaphtho-1,4-quinone

Bromine (25.4 g) was added to a stirred mixture of 6-methoxynaphtho-1,4-quinone (15.1 g, 0.08 mole) and anhydrous sodium acetate (13.3 g) in chloroform (125 ml) and the mixture stirred at room temperature for 3 days. After filtration the resulting liquid was evaporated to a dark solid, 23.2 g (84%) which after recrystallisation from petroleum ether (bp. 60—80°C) had mp 146—148°C. (Found; C, 38.33; H, 1.70; Br, 46.72; $C_{11}H_6Br_2O_3$ requires; C, 38.19; H, 1.75; Br, 46.19%).

b). 2-Amino-3-bromo-6(7)-methoxynaphtho-1,4-quinone

Dry ammonia was passed through a stirred, refluxing solution of 2,3-dibromo-6-methoxynaphtho-1,4-quinone (9.7 g, 0.028 mole) in N,N-dimethylformamide (80 ml) for 45 min. and the cooled mixture poured into water. The red solid which separated was filtered off, washed well with water and dried to give 7.34 g (93%) of product which had mp. ($EtOH/H_2O$) 184°C (dec).

c). 2-Acetamido-3-bromo-6(7)-methoxynaphtho-1,4-quinone

Concentrated sulphuric acid (2 drops) was added to a suspension of 2-amino-3-bromo-6(7)-methoxynaphtho-1,4-quinone (3.6 g, 0.0125 mole) in acetic anhydride (30 ml) and the dark mixture stirred for 15 min. After pouring into water an olive green solid separated which was filtered off and recrystallised from ethanol containing charcoal to give 3.4 g (84%) of a yellow solid of mp 229°C. (Found; C, 48.76; H, 2.90; N, 4.46; $C_{13}H_{10}BrNO_4$ requires; C, 48.17; H, 3.11; N, 4.32%).

d). 2-Acetamido-3-amino-6(7)-methoxynaphtho-1,4-quinone

Dry ammonia was passed for 1 hr. through a stirred, refluxing solution of 2-acetamido-3-bromo-6(7)-methoxynaphtho-1,4-quinone (5.1 g; 0.0158 mole) in nitrobenzene (50 ml) and the cooled solution filtered free of inorganic material and concentrated *in vacuo*. The red solid which separated was filtered off and washed with ether to give 2.5 g (61%) of material of mp 97°C (dec).

e). 4,9-Dihydro-4,9-dioxo-6-methoxy-1H-naphtho[2,3-d]-triazole

To a stirred suspension of 2-acetamido-3-amino-6(7)-methoxynaphtho-1,4-quinone (2.5 g; 0.0097 mole) in water (200 ml) was added a solution of sodium dithionite (4 g) in water (20 ml) in one portion. After a further 1 hr. the precipitated hydroquinone was filtered off and suspended in 2N hydroxhloric acid (70 ml) at 0°C. A solution of sodium nitrite (3 g) in water (20 ml) was added over 90 mins. and the mixture stirred overnight. The precipitated triazole was filtered off and recrystallised from acetone/water to give 0.88 g (38%) of derivative of mp 238°C (dec). (Found; C, 53.40; H, 3,75; N, 17.01; $C_{11}H_7N_3O_3$. $H_2O$ requires; C, 53.38; H, 3.67; N, 17.10%).

Example 4

4,9-Dihydro-4,9-dioxo-5-nitro-1H-naphtho-[2,3-d]-triazole

4,9-Dihydro-4,9-dioxo-1H-naphtho-[2,3-]-triazole (5 g, 0.025 mole) was dissolved in a mixture of concentrated sulphuric acid (25 ml) and fuming nitric acid (50 ml). The resulting solution was heated on a steam bath for 10—15 mins. and then poured onto ice (250 g). When all the ice had melted the solid was filtered off, washed with water (3 x 20 ml) and dried to yield a bright yellow solid (4.46 g, 74%), mp 227—35° (dec). $C^{13}NMR$ showed this to consist of a mixture of the 5- and 6-isomers in the approximate ratio 9:1. Multiple recrystallisation from acetone and then ethyl acetate yielded a yellow solid, mp 244—6° (dec) (0.640 g, 10.4%) consisting of the title compound containing 5% of the 6-isomer. (Found; C, 49.47; H, 1.48; N, 22.86; $C_{10}H_4N_4O_4$ requires; C, 49.20; H, 1.64; N, 22.95%).

Example 5

a). 2-Bromo-6-fluoro-3-hydroxynaphtho-1,4-quinone

A solution of bromine (12.5 g), in chloroform (50 ml) was added to a stirred suspension of 6-fluoro-3-hydroxynaphtho-1,4-quinone (12.5 g; 0.065 mole) in chloroform (50 ml) over 15 mins. During the addition the mixture became a clear dark red solution. Stirring was continued for a further 4 hours and a yellow solid which had been deposited as crystals was filtered off, washed with ice-cold chloroform and dried. Yield 13.95 g (80%), mp 183°C ($CHCl_3$).

b). 2-Amino-3-chloro-6(7)-fluoronaphtho-1,4-quinone

A solution of 2-bromo-6-fluoro-3-hydroxynaphtho-1,4-quinone (13.93 g; 0.052 mole) in dry benzene (200 ml) was refluxed with thionyl chloride (6.8 g, 0.057 mole) and D.M.F. (3 drops) for 3 hours. The dark red solution produced was cooled and evaporated to yield an orange solid which was recrystallised from chloroform. Analysis showed this solid to be a mixture of 2,3-dichloro-6-fluoro-naphtho-1,4-quinone and 2-bromo-3-chloro-6(7)-fluoronaphtho-1,4-quinone.

Dry ammonia was passed through a stirred solution of the above mixture (5.8 g) in nitrobenzene (75 ml) at room temperature for 3 hours. The mixture was filtered and the dark red filtrate was diluted with anhydrous ether. Dark red crystals were deposited. The crystals were collected, washed with anhydrous ether and dried. Yield 3.5 g mp 167° (d). Mass spec. $M^+$ 224.9991 corresponding to $C_{10}H_5ClNO_2$.

c). 2-Acetamido-3-chloro-6(7)-fluoronaphtho-1,4-quinone

Concentrated sulphuric acid (2 drops) was added to a suspension of 2-amino-3-chloro-6(7)-fluoronaphtho-1,4-quinone (3.5 g; 0.0155 mole) in acetic anhydride (25 ml). An immediate reaction took place and a yellow crystalline product was formed which was recrystallised from ethyl acetate. Yield 2.1 g (51%); mp 225—7° (EtOAc).

d). 2-Acetamido-3-amino-6(7)-fluoronaphtho-1,4-quinone

Dry ammonia was passed for 75 mins. through a stirred solution of 2-acetamido-3-chloro-6(7)-fluoronaphtho-1,4-quinone (2 g; 0.0079 mole) in nitrobenzene (20 ml) at 110°C. A dark red solid crystallised in the mixture. This was filtered off, extracted with chloroform, the extracts were combined and the solvent was evaporated to yield a red solid that was washed with dry ether. The product had mp 218—220°.

e). 4,9-Dihydro-4,9-dioxo-6-fluoro-1H-naphtho[2,3-d]-triazole

To a stirred suspension of 2-acetamido-3-amino-6(7)-fluoronaphtho-1,4-quinone (1.0 g, 0.004 mole) in water (150 ml) was added a solution of sodium dithionite (2.5 g) in water (10 ml) in one portion. After a further 1 hour the precipitated hydroquinone was filtered off and suspended in 2N HCl (50 ml) at 5°C. A solution of sodium nitrite (1.5 g) in water (15 ml) was added over 30 mins. and the mixture stirred overnight. The precipitated triazole was filtered off and recrystallised from acetone/water to give 0.55 g (63%) of derivative of mp 233° (dec), $\nu_{max}$(mull) 1690, 1600 $cm^{-1}$; $M^+$ 217.0296, $C_{10}H_4FN_3O_2$.

Example 6

4,9-Dihydro-6,7-dimethyl-4,9-dioxo-5-nitronaphtho[2,3-d]-triazole

4,9-Dihydro-6,7-dimethyl-4,9-dioxonaphthotriazole (0.4 g, 0.0018 mole) was dissolved in a mixture of concentrated sulphuric acid (4 ml) and fuming nitric acid (8 ml). The solution was heated on a steam bath for two hours and then poured onto ice (200 g). The iced mixture so obtained was left to stand overnight. The resulting solid was filtered off, washed with water (2 × 50 ml) and dried to give a pale yellow solid (380 mg, 80%) of mp 260—3°. This was recrystallised from ethanol to yield pale yellow crystals of mp 263—266°C (dec), $\nu_{max}$(mull) 3500, 3430, 1690 $cm^{-1}$, $\delta$(DMSO) 2.24 (3H, s); 2.55 (3H,s); 8.18 (1H,s), 1 mid field broad exchangeable proton. (Found; C, 53.10; H, 2.59; N, 20.56; $C_{12}H_8N_4O_4$ requires; C, 52.95; H, 2.96; N, 20.58%).

Example 7

(—)-Ephedrine salt of 4,9-Dihydro-6,7-dimethyl-4,9-dioxo-naphthol[2,3-d]-$\nu$-triazole

A solution of 4,9-dihydro-6,7-dimethyl-4,9-dioxo-naphtho[2,3-d]-$\nu$-triazole (454 mg, 0.002 mole) in methanol (20 ml) was added to a solution of (—)ephedrine (330 mg, 0.002 mole) in methanol (10 ml) in one portion. After standing for 15 mins. the solution remained clear although the colour had darkened. The solvent was removed *in vacuo* and the brown solid recrystallised from chloroform/petrol (60—80°) to give 550 mg (63%) of the above named salt, mp 252°C, $\nu_{max}$(mull) 3350—2400 broad, 1665, 1600, 1390, 1260, 980 $cm^{-1}$. $\delta$(DMSO) 0.97 (3H, d); 2.36 (6H, s); 2.54 (1H, quartet); 2.70 (3H, s); 5.14 (1H, d); 7.42 (5H, s); 7.88 (3H, s) and exchangeables. (Found; C, 64.18; H, 6.46; N, 13.28; $C_{22}H_{24}N_4O_3H_2O$ requires; C, 64.45; H, 6.39; N, 13.65%).

Example 8

4,9-Dihydro-4,9-dioxo-1H-naphtho[2,3-d]-triazole

A solution of sodium dithionite (2.5 g) in water (10 ml) was added to a stirred suspension of 2-acetylamino-3-amino-1,4-naphthoquinone (1.5 g) in water (150 ml) and the mixture stirred at room temperature until the solid had decolourised (1 hr.). The solid was filtered off, dissolved in water (25 ml) containing concentrated hydrochloric acid (3 ml), decolourised with charcoal and diluted to 50 ml by addition of water. A solution of sodium nitrite (10 ml of 10%) was added to cooled (0°C) stirred solution and after 1 hr. at 0° off-white solid was filtered off. Recrystallisation from acetone-water gave 0.62 g (44%) of the title compound of mp 250°C (dec) (Lit. mp 240—245°C Fieser and Martin loc. cit.). $\nu_{max}$(mull) 1690 $cm^{-1}$. (Found; C, 60.05; H, 2.81; N, 20.90; $C_{10}H_5N_3O_2n$ requires; C, 60.30; H, 2.53; N, 21.01%).

Example 9

Ammonium Salt of 4,9-Dihydro-4,9-dioxo-5-nitro-naphtho[2,3-d]triazole

4,9-dihydro-4,9-dioxo-5-nitronaphthotriazole (0.244 g, 0.001 mole) was suspended in ammonia solution (1:1 water/880 ammonia) and warmed on a steam bath for 30 minutes. The mixture became reddish and was cooled to room temperature. The insoluble solid was filtered off and dried to yield 0.21 g (80%) of a pink solid whose NMR spectrum showed it to be the title compound ($d^6$.DMSO, typical 5-substitution pattern multiplet $\delta$ 8.0—8.5; broad exchangeable peak 5.7,4H). The compound had no melting point but charred at *circa* 235°C. (Found; C, 45.51; H, 2.44; N, 26.18; $C_{10}H_7N_5O_4$ requires; C, 45.98; H, 2.70; N, 26.81).

Example 10

Sodium Salt of 4,9-Dihydro-6,7-dimethyl-4,9-dioxonaphtho[2,3-d]triazole

a) A suspension of the parent triazole (1.33 g) in water (50 ml) was treated with 2.5N sodium hydroxide solution until the pH of the solution was 6.5. The resulting yellow solution was filtered and evaporated to dryness. The residual yellowish solid was recrystallised from ethanol to yield a buff solid which turned yellow on drying. Yield 1.433 g mp 360°. Anhydrous material was obtained by drying in vacuum

at 110° over phosphorus pentoxide when the material looses its crystallinity (Found; Na: 8.89%, $C_{12}H_8N_3O_2$ Na requires; 9.23%).

b) 4,9-Dihydro-6,7-dimethyl-4,9-dioxonaphtho[2,3-d]triazole (1.0 g) was suspended in water (50 ml) and the pH brought to 6.8 by the addition of 5N sodium hydroxide solution. The resulting solution was evaporated to dryness *in vacuo.* The residue was shaken with ethanol (20 ml) and again evaporated to dryness, this ethanol treatment was repeated. The solid was now dissolved in a minimum of boiling ethanol and the resulting solution cooled to 5°C. A few needles slowly formed, these were filtered off, rinsed with ethanol and dried to yield 0.21 g of bright yellow needles. The NMR spectrum of this material (in $d^6$DMSO) contained a peak corresponding to 10H (after substracting the solvent contribution) which exchanged with $D_2O$. Microanalysis was also consistant with the presence of a pentahydrate (Found; C, 42.91, H, 5.14, N, 12.41, $C_{12}H_8N_3O_2Na.5H_2O$ requires C, 42.48, H, 5.35, N, 12.39).

c) Crude 4,9-dihydro-6,7-dimethyl-4,9-dioxonaphtho[2,3-d]triazole sodium salt was prepared as in b) above. The residue from evaporation of the solution for the third time was dried at 0.2 mm/Hg for about 5 minutes and then dissolved in the minimum of boiling methanol. On cooling bright yellow needles were deposited from the solution, these were filtered off and dried. NMR suggests these are anhydrous.

Passive Cutaneous Anaphylaxis (PCA)

Serum containing heat labile homocytotropic antibody was raised in rats to crystallised ovalbumin XOA by the method of Mota (I. Mota, Immunology, *7,* 681 [1964]) using Bordettela pertussis vaccine as adjuvant.

Passive cutaneous anaphylaxis (PCA) was carried out by a method based on that of Ovary and Bier, (A. Ovary and O. G. Bier, Proc. Soc. Exp. Biol. Med. *81*, 584, [1952]) as modified by Goose and Blair. (Immunology *16,* 749 (1969).

Male Wistar rate of 250—300 g were given 0.01 ml of each of six twofold serial dilutions of pooled antiserum in 0.9% saline injected intradermally into separate sites on their shaved backs. Later (72 hrs.) the animals were challenged by intravenous injection of 0.3 ml of a 1% solution of ovalbumin in an isoptonic solution of saline buffered with 0.05 M, pH 7.2, Sorenson Buffer (PBS), mixed with 0.2 ml of a 5% solution of Pontamine Sky Blue (6BX C.I. 24410, Raymond A. Lamb, London) in isotonic saline. The rats were killed after 20 min and the diameter of the blue wheals at the antibody injection sites was measured on the outer surface of the skin. The starting dilution of the serum was adjusted so that there was no response, after challenge, at the injection site of the highest dilution and a maximum response at the lowest dilutions. Typically six twofold serial dilutions of the serum from 1/4 to 1/128 were used.

Compounds were tested for their ability to reduce the diameter of the wheals at those intradermal sites which in control animals gave less than maximum response. Each dose of the compound was administered to six rats at a measured time prior to intravenous challenge with ovalbumin. Control groups of six rats were given the same volume of carrier fluid (0.2 ml, 100 $g^{-1}$) at the same time prior to challenge.

The results were calculated as follows: % inhibition of PCA = 100 (1 — a/b) where a = the sum of the diameters of the wheals produced in the test animal at the sites of antibody dilutions as used in control groups and b = the mean sum of the diameters of the wheals produced in the control group of animals at those antibody sites where at least five out of six of the animals gave less than maximum response. A typical variation in the control group of animals was SEM± 6%.

| Example | Route | Carrier Fluid | Time* (mins) | Dose mg /kg | % Inhib PCA |
|---|---|---|---|---|---|
| 1(d) | S.C. | PBS with $NaHCO_3$ | 5 | 0.1 | 30 |
| | | | 5 | 0.2 | 54 |
| | | | 5 | 0.4 | 61 |
| | | | 5 | 0.8 | 86 |
| 2(d) | S.C. | PBS with $NaHCO_3$ | 10 | 20 | 73 |
| | | | 30 | 20 | 57 |
| 3(e) | S.C. | PBS with $NaHCO_3$ | 10 | 20 | 61 |
| | | | 30 | 20 | 34 |
| 4 | S.C. | PBS with $NaHCO_3$ | 10 | 10 | 44 |
| | | | 30 | 10 | 19 |
| 5(e) | S.C. | PBS with $NaHCO_3$ | 10 | 20 | 90 |
| | | | 30 | 20 | 27 |
| 6 | S.C. | PBS with $NaCHO_3$ | 10 | 1 | 66 |
| | | | 30 | 1 | 28 |
| 8 | S.C. | PBS with $NaCHO_3$ | 5 | 1 | 39 |
| | | | 5 | 2 | 82 |

* Time between administration of compound and antigen challenge.

Acute toxicity: no toxic symptoms were observed with any of the compounds while carrying out the tests described above.

Toxicity: Oral $LD_{50}$ of the compound of example 1(d) in Charles River Sprage Dawley CD strain Male Rats: 980 $mgkg^{-1}$, $LD_{50}$ data confidence limits: 95%, 548–1,413 $mgkg^{-1}$.

**Claims**

1. A pharmaceutical composition adapted for administration to human beings, comprising a compound of formula (I), or a pharmaceutically acceptable salt thereof:

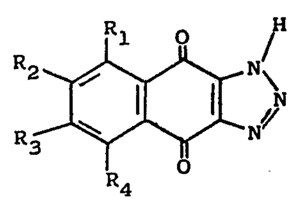

(I)

wherein $R_1$, $R_2$, $R_3$ and $R_4$ which may be the same or different, represent hydrogen, halogen, nitro, $C_{1-6}$ alkyl or $C_{1-6}$ alkoxy, or any adjacent two of $R_1$ to $R_4$ taken together represent an alkylene group containing from 3 to 5 carbon atoms or a 1,4-buta-1,3-dienylene group and a pharmaceutically acceptable carrier.

2. A composition according to claim 1, wherein $R_1$, $R_2$, $R_3$ and $R_4$ in the compound of formula (I) represent hydrogen, methyl, ethyl, propyl, methoxy, ethoxy, propoxy, fluorine or chlorine.

3. A composition according to claim 1 or 2, wherein at least one of $R_1$ to $R_4$ in the compound of formula (I) is hydrogen.

4. A composition according to claim 1, 2 or 3, wherein two of $R_1$ to $R_4$ in the compound of formula (I) are hydrogen.

5. A composition according to any one of the claims 1 to 4, wherein $R_1$ and $R_4$ are both hydrogen in the compound of formula (I).

6. A composition according to claim 5, wherein $R_2$ and $R_3$ which may be the same or different represent methyl, ethyl or *n*-propyl in the compound of formula (I).

7. A compound of formula (I)', or a pharmaceutically acceptable salt thereof:

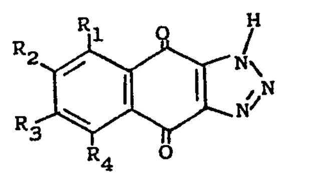

(I)

wherein $R_1$ and $R_4$ are the same or different and represent hydrogen, halogen, nitro, $C_{1-6}$ alkyl or $C_{1-6}$ alkoxy; and $R_2$ and $R_3$ are the same or different and represent halogen, nitro, $C_{1-6}$ alkyl or $C_{1-6}$ alkoxy; or $R_2$ and $R_3$ taken together represent an alkylene group containing from 3 to 5 carbon atoms or a buta-1,3-dienylene group.

8. A compound according to claim 7, wherein $R_1$ and $R_4$ represent hydrogen, methyl, ethyl, propyl, methoxy, ethoxy, propoxy, fluorine or chlorine, and $R_2$ and $R_3$ represent methyl, ethyl, propyl, methoxy, ethoxy, propoxy, fluorine or chlorine.

9. A compound according to claim 7 or 8, wherein at least one of $R_1$ and $R_4$ are hydrogen.

10. A compound according to any of the claims 7 to 9, wherein $R_1$ and $R_4$ are both hydrogen.

11. A compound according to claim 10, wherein $R_2$ and $R_3$ which may be the same or different represent methyl, ethyl or *n*-propyl.

12. A compound according to claim 7 which is 4,9-Dihydro-6,7-dimethyl-4,9-dioxo-1H-naphtho-[2,3-d]-triazole.

13. A compound according to claim 7 which is a pharmaceutically acceptable salt of 4,9-dihydro-6,7-dimethyl-4,9-dioxo-1H-naphtho-[2,3-d]-triazole.

14. A compound according to claim 7 which is 4,9-Dihydro-6,7-dimethyl-4,9-dioxo-1H-naphtho-[2,3-d]-triazole sodium salt.

15. A pharmaceutical composition comprising a compound according to claim 12, 13 or 14, and a pharmaceutically acceptable carrier.

16. A compound of the formula (I) as defined in any one of the claims 1 to 14 for use in the treatment of allergies.

17. A method for preparing a compound of formula (I)' according to claim 7 which method comprises oxidizing a naphthotriazole of formula (II):

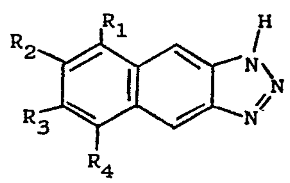

(II)

wherein $R_1$, $R_2$, $R_3$ and $R_4$ are as defined in claim 7 with a powerful oxidizing agent, and thereafter optionally converting the compound of formula (I)' into a pharmaceutically acceptable salt.

18. A method for preparing a compound of the formula (I)' according to claim 7 which method comprises deamination of a 2-amino naphtho triazole (III):

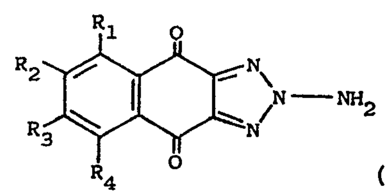

(III)

wherein $R_1$, $R_2$, $R_3$ and $R_4$ are as defined in claim 7 with nitrous acid, and thereafter optionally converting the compound of formula (I)' so formed into a pharmaceutically acceptable salt.

19. A method for preparing a compound of the formula (I)' according to claim 7, which method comprises reacting a compound of formula (IV):

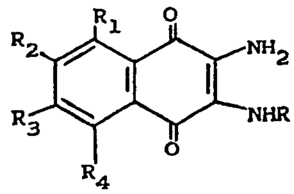

(IV)

wherein $R_1$, $R_2$, $R_3$ and $R_4$ are as defined in claim 7 and R is hydrogen or an acyl group, with nitrous acid, and thereafter optionally converting the compound of formula (I)' so formed into a pharmaceutically acceptable salt.

20. A method for preparing a compound of the formula (I)' according to claim 7 which method comprises reacting a hydroquinone (IVa):

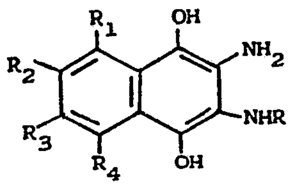

(IVa)

wherein R and $R_1$ to $R_4$ are as defined with reference to formula (IV) with nitrous acid, and thereafter optionally converting the compound of formula (I)' so formed into a pharmaceutically acceptable salt.

21. A method according to claim 19 or claim 20, wherein the nitrous acid is generated *in situ* from an alkali metal nitrite and an acid.

22. A method as claimed in claim 21, wherein the alkali metal nitrite is sodium nitrite and the acid is hydrochloric acid.

23. A method for preparing a compound of formula (Ia):

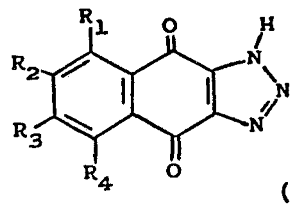

(Ia)

or a pharmaceutically acceptable salt thereof; wherein $R_1$, $R_2$, $R_3$ and $R_4$ are as defined in claim 7 provided that at least one of $R_1$ to $R_4$ represent nitro which method comprises nitrating a compound of formula (Ib):

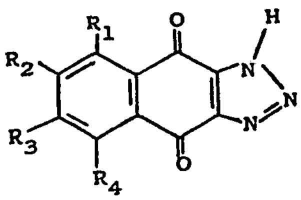

(Ib)

wherein $R_1$, $R_2$, $R_3$ and $R_4$ are as defined in claim 7 provided that at least one of $R_1$ to $R_4$ represents hydrogen, and thereafter converting the compound of formula (Ia) into a pharmaceutically acceptable salt.

**Revendications**

1. Composition pharmaceutique adaptée à l'administration à l'homme, caractérisée en ce qu'elle comprend un composé de formule (I) ou un sel pharmaceutiquement acceptable d'un tel composé:

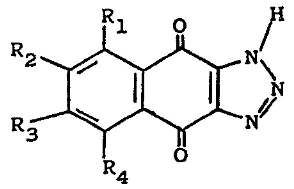

(I)

où $R_1$, $R_2$, $R_3$ et $R_4$, qui peuvent être identiques ou différents, représentent de l'hydrogène, un halogène, un groupe nitro, alcoyle en $C_{1-6}$ ou alcoxy en $C_{1-6}$, ou deux groupes quelconques adjacents parmi les groupes $R_1$ à $R_4$ représentent, pris ensemble, un groupe alcoylène contenant de 3 à 5 atomes de carbone ou un groupe, 1,4-buta-1,3-diénylène, et un excipient pharmaceutiquement acceptable.

2. Composition suivant la revendication 1, caractérisée en ce que $R_1$, $R_2$, $R_3$ et $R_4$ dans le composé de formule (I) représentent de l'hydrogène, des groupes méthyle, éthyle, propyle, méthoxy, éthoxy, propoxy, du fluor ou du chlore.

3. Composition suivant la revendication 1 ou 2 caractérisée en ce qu'au moins l'un des radicaux $R_1$ à $R_4$ dans le composé de formule (I) est de l'hydrogène.

4. Composition suivant la revendication 1, 2 ou 3 caractérisée en ce que deux des groupes $R_1$ à $R_4$ dans le composé de formule (I) sont de l'hydrogène.

5. Composition suivant l'une quelconque des revendications 1 à 4, caractérisée en ce que $R_1$ et $R_4$ sont tous les deux de l'hydrogène dans le composé de formule (I).

6. Composition suivant la revendication 5, caractérisée en ce que $R_2$ et $R_3$, qui peuvent être identiques ou différents, représentent les groupes méthyle, éthyle ou n-propyle dans le composé de formule (I).

7. Composé de formule (I)' ou sel pharmaceutiquement acceptable de ce composé.

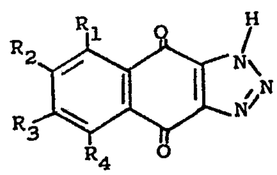

(I)'

où $R_1$ et $R_4$ sont identiques ou différents et représentent de l'hydrogène, un halogène, un groupe nitro, alcoyle en $C_{1-6}$ ou alcoxy en $C_{1-6}$; et $R_2$ et $R_3$ sont identiques ou différents et représentent un halogène, un groupe nitro, alcoyle en $C_{1-6}$ ou alcoxy en $C_{1-6}$; ou $R_2$ et $R_3$ pris ensemble représentent un group alcoylène contenant de 3 à 5 atomes de carbone ou un groupe buta-1,3-diénylène.

8. Composé suivant la revendication 7, caractérisé en ce que $R_1$ et $R_4$ représentent de l'hydrogène, des groupes méthyle, éthyle, propyle, méthoxy, éthoxy, propoxy, du fluor ou du chlore, et $R_2$ et $R_3$ représentent des groupes méthyle, éthyle, propyle, méthoxy, éthoxy, propoxy, du fluor ou du chlore.

9. Composé suivant la revendication 7 ou 8, caractérisé en ce qu'au moins l'un des radicaux $R_1$ et $R_4$ est de l'hydrogène.

10. Composé suivant l'une quelconque des revendications 7 à 9, caractérisé en ce que $R_1$ et $R_4$ sont tous les deux de l'hydrogène.

11. Composé suivant la revendication 10, caractérisé en ce que $R_2$ et $R_3$, qui peuvent être identiques ou différents, représentent des groupes méthyle, éthyle ou n-propyle.

12. Composé suivant la revendication 7, formé par le 4,9-dihydro-6,7-diméthyl-4,9-dioxo-1H-naphto[2,3-d]-triazole.

13. Composé suivant la revendication 7, formé par un sel pharmaceutiquement acceptable de 4,9-dihydro-6,7-diméthyl-4,9-dioxo-1H-naphto-[2,3-d]triazole.

14. Composé suivant la revendication 7, formé par le sel de sodium de 4,9-dihydro-6,7-diméthyl-4,9-dioxo-1H-naphto[2,3-d]-triazole.

15. Composition pharmaceutique, caractérisée en ce qu'elle comprend un composé suivant la revendication 12, 13 ou 14 et un excipient pharmaceutiquement acceptable.

16. Composé de formule (I) tel que défini dans l'une quelconque des revendications 1 à 14 pour l'utilisation pour le traitement d'allergies.

17. Procédé de préparation d'un composé de formule (I)' suivant la revendication 7, caractérisé en ce qu'on oxyde un naphtotriazole de formule (II)

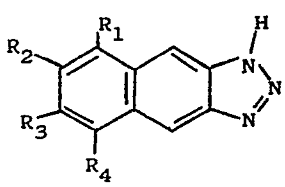

(II)

où $R_1$, $R_2$, $R_3$ et $R_4$ sont tels que définis dans la revendication 7, au moyen d'un agent d'oxydation puissant, et on transforme ensuite facultativement le composé de formule (I)' en un sel pharmaceutiquement acceptable.

18. Procédé de préparation d'un composé de formule (I)' suivant la revendication 7, caractérisé en ce qu'il comprend la désamination de 2-amino-naphto-triazole (III):

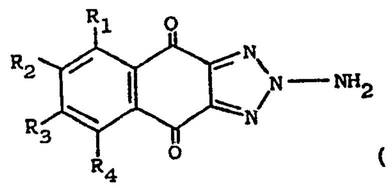

(III)

où $R_1$, $R_2$, $R_3$ et $R_4$ ont les significations indiquées dans la revendication 7, par de l'acide nitreux et on transforme ensuite facultativement le composé de formule (I)' ainsi formé en un sel pharmaceutiquement acceptable.

19. Procédé de préparation d'un composé de formule (I)' suivant la revendication 7, caractérisé en ce qu'on fait réagir un composé de formule (IV);

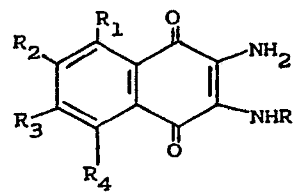

(IV)

où $R_1$, $R_2$, $R_3$ et $R_4$ ont les significations indiquées dans la revendication 7 et R est de l'hydrogène ou un groupe acyle, avec de l'acide nitreux, et on transforme ensuite facultativement le composé de formule (I)' ainsi formé en un sel pharmaceutiquement acceptable.

20. Procédé de préparation d'un composé de formule (I)' suivant la revendication 7, caractérisé en ce qu'on fait réagir une hydroquinone (IVa):

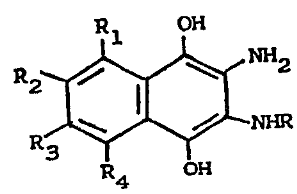

(IVa)

où R et $R_1$ à $R_4$ ont les significations indiquées en ce qui concerne la formule (IV() avec de l'acide nitreux, et on transforme ensuite facultativement le composé de formule (I)' ainsi formé en un sel pharmaceutiquement acceptable.

21. Procédé suivant la revendication 19 ou 20, caractérisé en ce que l'acide nitreux est produit in situ à partir d'un nitrite de métal alcalin et d'un acide.

22. Procédé suivant la revendication 21, caractérisé en ce que le nitrite de métal alcalin est du nitrite de sodium et l'acide est de l'acide chlorhydrique.

23. Procédé de préparation d'un composé de formule (Ia)

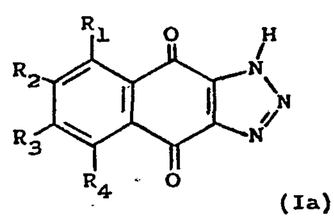

(Ia)

ou d'un sel pharmaceutiquement acceptable de ce composé, où $R_1$, $R_2$, $R_3$ et $R_4$ ont les significations indiquées dans la revendication 7, à condition qu'au moins l'un des radicaux $R_1$ à $R_4$ représente un groupe nitro, caractérisé en ce qu'on effectue la nitration d'un composé de formule (Ib):

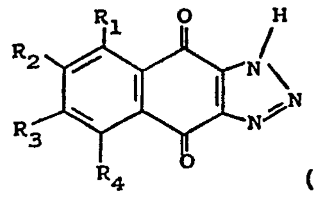

(Ib)

où $R_1$, $R_2$, $R_3$ et $R_4$ ont les significations indiquées dans la revendication 7, à condition qu'au moins l'un des radicaux $R_1$ à $R_4$ représente de l'hydrogène, et on transforme ensuite le composé de formule (Ia) en un sel pharmaceutiquement acceptable.

**Patentansprüche**

1. Pharmazeutische Zusammensetzung, angepaßt zur Verabreichung an Menschen, enthaltend eine Verbindung der Formel I oder ein pharmakologisch verträgliches Salz derselben

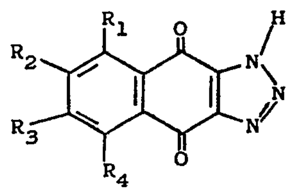

(I)

in der $R_1$, $R_2$, $R_3$ und $R_4$, die gleich oder verschieden sein können, Wasserstoff- oder Halogenatome, Nitro-, $C_{1-6}$-Alkyl- oder $C_{1-6}$-Alkoxyreste bedeuten oder zwei benachbarte Reste aus der Gruppe $R_1$ bis $R_4$ zusammen einen Alkylenrest mit 3 bis 5 Kohlenstoffatomen oder einen 1,4-Buta-1,3-dienylenrest bilden, und einen pharmakologisch verträglichen Trägerstoff.

2. Zusammensetzung nach Anspruch 1, dadurch gekennzeichnet, daß $R_1$, $R_2$, $R_3$ und $R_4$ in der Verbindung der Formel I Wasserstoffatome, Methyl-, Äthyl-, Propyl-, Methoxy-, Äthoxy- oder Propoxyreste, Fluor- oder Chloratome sind.

3. Zusammensetzung nach Anspruch 1 oder 2, dadurch gekennzeichnet, daß mindestens einer der Reste $R_1$ bis $R_4$ in der Verbindung der Formel I ein Wasserstoffatom ist.

4. Zusammensetzung nach Anspruch 1, 2 oder 3, dadurch gekennzeichnet, daß zwei der Reste $R_1$ bis $R_4$ in der Verbindung der Formel I Wasserstoffatome sind.

5. Zusammensetzung nach einem der Ansprüche 1 bis 4, dadurch gekennzeichnet, daß $R_1$ und $R_4$ in der Verbindung der Formel I beide Wasserstoffatome sind.

6. Zusammensetzung nach Anspruch 5, dadurch gekennzeichnet, daß $R_2$ und $R_3$ in der Verbindung der Formel I, die gleich oder verschieden sein können, Methyl-, Äthyl- oder n-Propylreste bedeuten.

7. Eine Verbindung der Formel I' oder ein pharmakologisch verträgliches Salz derselben

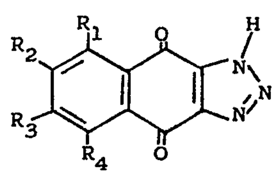

(I)'

in der $R_1$ und $R_4$ gleich oder veschieden sind und Wasserstoff- oder Halogenatome, Nitro-, $C_{1-6}$-Alkyl- oder $C_{1-6}$-Alkoxyreste bedeuten und $R_2$ und $R_3$ gleich oder verschieden sind und Halogenatome, Nitro-, $C_{1-6}$-Alkyl- oder $C_{1-6}$-Alkoxyreste bedeuten oder $R_2$ und $R_3$ zusammen einen Alkylenrest mit 3 bis 5 Kohlenstoffatomen oder einen Buta-1,3-dienylenrest bilden.

8. Eine Verbindung nach Anspruch 7, dadurch gekennzeichnet, daß $R_1$ und $R_4$ Wasserstoff-, Methyl-, Äthyl-, Propyl-, Methoxy-, Äthoxy- oder Propoxyreste, Fluor- oder Chloratome bedeuten und $R_2$ und $R_3$ Methyl-, Äthyl-, Propyl-, Methoxy-, Äthoxy- oder Propoxyreste oder Fluor- oder Chloratome bedeuten.

9. Eine Verbindung nach Anspruch 7 oder 8, dadurch gekennzeichnet, daß mindestens einer der Reste $R_1$ und $R_4$ ein Wasserstoffatom ist.

10. Eine Verbindung nach einem der Ansprüche 7 bis 9, dadurch gekennzeichnet, daß $R_1$ und $R_4$ beide Wasserstoffatome sind.

11. Eine Verbindung nach Anspruch 10, dadurch gekennzeichnet, daß $R_2$ und $R_3$, die gleich oder verschieden sein können, Methyl-, Äthyl- oder n-Propylreste bedeuten.

12. Eine Verbindung nach Anspruch 7, dadurch gekennzeichnet, daß sie 4,9-Dihydro-6,7-dimethyl-4,9-dioxo-1H-naphtho[2,3-d]-triazol ist.

13. Eine Verbindung nach Anspruch 7, dadurch gekennzeichnet, daß sie ein pharmakologisch verträgliches Salz von 4,9-Dihydro-6,7-dimethyl-4,9-dioxo-1H-naphtho[2,3-d]-triazol ist.

14. Eine Verbindung nach Anspruch 7, dadurch gekennzeichnet, daß sie 4,9-Dihydro-6,7-dimethyl-4,9-dioxo-1H-naphtho[2,3-d]-triazol-natriumsalz ist.

15. Pharmazeutische Zusammensetzung, enthaltend eine Verbindung nach Anspruch 12, 13 oder 14 und einen pharmakologisch verträglichen Trägerstoff.

16. Eine Verbindung der Formel I, wie in einem der Ansprüche 1 bis 14 definiert, zur Verwendung in der Behandlung von Allergien.

17. Verfahren zur Herstellung einer Verbindung der Formel I′ gemäß Anspruch 7, die Maßnahme einschließend, daß ein Naphtho-triazol der Formel II

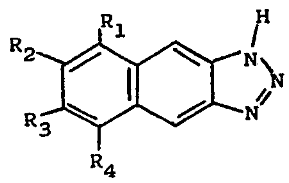

(II)

in der $R_1$, $R_2$, $R_3$ und $R_4$ wie in Anspruch 7 definiert sind, mit einem starken Oxidationsmittel oxidiert und anschließend gegebenenfalls die Verbindung der Formel I′ in ein pharmakologisch verträgliches Salz umgewandelt wird.

18. Verfahren zur Herstellung einer Verbindung der Formel I′ gemäß Anspruch 7, die Maßnahme einschließend, daß ein 2-Amino-naphtho-triazol der Formel III

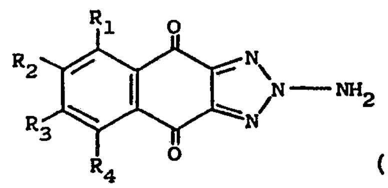

(III)

in der $R_1$, $R_2$, $R_3$ und $R_4$ wie in Anspruch 7 definiert sind, mit salpetriger Säure desaminiert und anschließend gegebenenfalls die so gebildete Verbindung der Formel I′ in ein pharmakologisch verträgliches Salz umgewandelt wird.

19. Verfahren zur Herstellung einer Verbindung der Formel I′ gemäß Anspruch 7, die Maßnahme einschließend, daß eine Verbindung der Formel IV

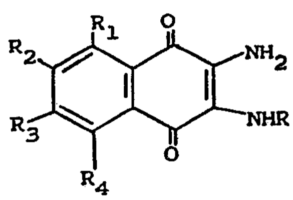

(IV)

in der $R_1$, $R_2$, $R_3$ und $R_4$ wie in Anspruch 7 definiert sind und R ein Wasserstoffatom oder ein Acylrest ist, mit salpetriger Säure umgesetzt und anschließend gegebenenfalls die so gebildete Verbindung der Formel I′, in ein pharmakologisch verträgliches Salz umgewandelt wird.

20. Verfahren zur Herstellung einer Verbindung der Formel I′ gemäß Anspruch 7, die Maßnahme einschließend, daß ein Hydrochinon der Formel IVa

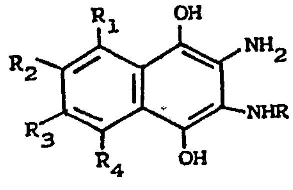

(IVa)

in der R und $R_1$ bis $R_4$ wie in Bezug auf Formel IV definiert sind, mit salpetriger Säure umgesetzt und anschließend gegebenenfalls die so gebildete Vernbindung der Formel I' in ein pharmakologisch verträgliches Salz umgewandelt wird.

21. Verfahren nach Anspruch 19 oder 20, wobei die salpetrige Säure in situ aus einem Alkalimetallnitrit und einer Säure hergestellt wird.

22. Verfahren nach Anspruch 21, wobei das Alkalimetallnitrit Natriumnitrit und die Säure Salzsäure ist.

23. Verfahren zur Herstellung einer Verbindung der Formel Ia oder eines pharmakologisch verträglichen Salzes derselben

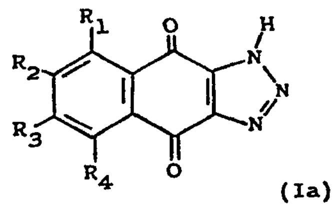

(Ia)

in der $R_1$, $R_2$, $R_3$ und $R_4$ wie in Anspruch 7 definiert sind mit der Maßgabe, daß mindestens einer der Reste $R_1$ bis $R_4$ eine Nitrogruppe ist, die Maßnahme einschließend, daß eine Verbindung der Formel Ib

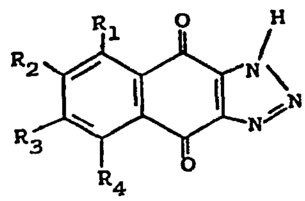

(Ib)

in der $R_1$, $R_2$, $R_3$ und $R_4$ wie in Anspruch 7 definiert sind mit der Maßgabe, daß mindestens einer der Reste $R_1$ bis $R_4$ ein Wasserstoffatom ist, nitriert und anschließend gegebenenfalls die Verbindung der Formel Ia in ein pharmakologisch verträgliches Salz umgewandelt wird.